Europäisches Patentamt

European Patent Office

Office européen des brevets

(11) Publication number: **0 413 307 A1**

# EUROPEAN PATENT APPLICATION

(21) Application number: 90115575.4

(22) Date of filing: 14.08.90

(51) Int. Cl.⁵: **C12P 7/62**, C12P 19/44, C12P 7/64, C12P 19/02, C12N 9/20, C07H 15/04

(30) Priority: 15.08.89 JP 210495/89
02.05.90 JP 116328/90

(43) Date of publication of application:
20.02.91 Bulletin 91/08

(84) Designated Contracting States:
AT BE CH DE DK ES FR GB GR IT LI LU NL SE

(71) Applicant: **LION CORPORATION**
3-7, Honjo 1-chome
Sumida-ku Tokyo(JP)

(72) Inventor: **Miyake, Hiroshi**
**No. 24-16, Yagigatani 1-chome**
**Funabashi-shi, Chiba-ken(JP)**
Inventor: **Kurahashi, Midori**
**No. 14-19-205, Higashi-cho 3-chome**
**Houya-shi, Tokyo(JP)**
Inventor: **Toda, Haruhiko**
**No. 15-9, Maeno-cho 5-chome, Itabashi-ku**
**Tokyo(JP)**
Inventor: **Kitano, Kyozo**
**No. 1-3-402, Akitsu 2-chome**
**Narashino-shi, Chiba-ken(JP)**
Inventor: **Mori, Takao, Room No. 405**
**Akashi-cho, Flats, No. 7-20, Akashi-cho**
**Chuo-ku, Tokyo(JP)**

(74) Representative: **Vossius & Partner**
**Siebertstrasse 4 P.O. Box 86 07 67**
**D-8000 München 86(DE)**

(54) Process for producing saccharide fatty acid monoesters.

(57) A process for producing a saccharide fatty acid monoester comprises reacting an immobilized thermostable lipase on a mixture comprising:
(A) one or more of acyl donors selected from saturated and unsaturated fatty acids with 6 to 22 carbon atoms, lower alkyl esters of said fatty acids and glycerol esters of said fatty acids,
(B) one or more of saccharides selected from monosaccharides with 5 to 7 carbon atoms, disaccharides composed of hexose, sugar alcohols with 4 to 6 carbon atoms, ethers of monosaccharides with 5 to 7 carbon atoms and monohydric alcohols and ethers of disaccharides composed of hexose and monohydric alcohols, and
(C) one or more of alcohols selected from secondary alcohols and tertiary alcohols.

# PROCESS FOR PRODUCING SACCHARIDE FATTY ACID MONOESTERS

The present invention concerns a process for producing saccharide fatty acid monoesters capable of synthesizing saccharade fatty acid monoesters at a high synthesis rate and high content by way of an enzymatic reaction using a lipase.

It has been known that lipase is an enzyme of hydrolyzing fats or esters of higher fatty acids, and it has also been known that lipase causes a reverse reaction of the hydrolysis under an appropriate condition to synthesize esters or conduct ester-exchange reactions.

However, if the enzymatic reaction is carried out in an aqueous solution, ester hydrolysis as the reverse reaction is taken place preferentially in the case of an ester synthesis reaction. Also in the case of the ester-exchange reaction, ester hydrolysis for the starting material and the products are caused to reduce the reaction ratio.

For avoiding such ester hydrolysis, it has been proposed to conduct the ester synthesis or the ester-exchange reaction in a substantially anhydrous organic solvent by means of an enzymatic process (refer, for example, to Japanese Patent Laid-Open Nos. 61-268192 and 62-10779; J. Am. Chem. Soc., 108, 5638 (1986) ; J. Am. Chem. Soc., 110, 584 (1988)). In this case, Japanese Patent Laid-Open No. 61-268192 uses an alkaline lipase derived from microorganisms, and Japanese Patent Laid-Open No. 62-10779 uses a lipase derived through variation from Candida cylindracea. Further, in each of the above-mentioned literatures, improvement for the reaction rate is attempted by using an active ester of a complicate structure.

However, since a special substrate is used for increasing the monoester content in the known method, for example, in the method as described in the above-mentioned literature, no sufficient reaction rate can be provided by means of usual fatty acids or lower alcohol esters thereof. Further, monoesters can not be obtained selectively by the method described in Japanese Patent Laid-Open No. 61-268192.

In addition, it has been reported a method of esterifying saccharides through a chemical synthesis process by using an acylating agent such as an acid chloride (Chem. Pharm. Bull, 27 (11), 2661 (1979)). However, a great amount of polyesters are by-produced even if the acylating agent and saccharides are used each in an equimolar ratio and monoesters can not be obtained selectively by this method.

On the other hand, as a method of preparing a fatty acid ester of an alkyl glucoside; it has been proposed a chemical synthesis process of reacting glucoside of hexose with an acyl donor such as a fatty acid or a fatty acid derivative under the presence of dehydrative coupling agent or a basic compound (U.S. Patent No. 4716152).

However, it is difficult in the chemical synthesis process to obtain only a monoester at a high selectivity and it also involves a drawback of using a great amount of an expensive dehydrative coupling agent or using a fatty acid derivative (acid chloride, etc.) which is difficult to be used industrially.

Meanwhile, an enzymatic production process has also been noted in recent years and there has been proposed a method of reacting a fatty acid on an alkyl glucoside having $C_3$ or higher alkyl group under the presence of water of less than a predetermined amount and a fatty acid decomposing enzyme (Japanese Patent Laid-Open No. 2-9436). Further, it has also been proposed a method of reacting a hydrolase on a mixture of an alkyl glucoside having $C_2$ - $C_4$ alkyl group and a fatty acid or lower alcohol ester of fatty acid (WO 89/01480).

However, in the former method, use of a lower alkyl glucoside having an alkyl group such as methyl or ethyl has no effect. The latter method involves a disadvantage that it is not effective to methyl glucoside. In addition, it is difficult in any of the methods to obtain a fatty acid ester of alkyl glucoside having a monoester content at a sufficiently high reaction rate. In particular, if the amount of the acyl donor is increased relative to the alkyl glucoside for increasing the reaction efficiency, polysubstitutes such as diesters are by-produced in a great amount.

Accordingly, it can not be obtained saccharide fatty acid esters having high monoester content at a sufficiently high reaction rate by the conventional method in a case of reacting usual fatty acids or lower alcohol esters thereof and saccharides.

It is, accordingly, an object of the present invention to provide a process for producing a saccharide fatty acid monoester capable of synthesizing saccharide fatty acid monoesters in a short period of time and at a high synthesis ratio with high or increased content in the reaction products by using an enzymatic reaction of lipase.

The present invention provides a process for producing a saccharide fatty acid monoester which comprises reacting an immobilized thermostable lipase on a mixture comprising:

A: one or more of acyl donors selected from saturated and unsaturated fatty acids with to 6 to 22 carbon

2

EP 0 413 307 A1

atoms, lower alkyl esters of such fatty acids, and glycerol esters of such fatty acids,

B: one or more of saccharides selected from monosaccharides with 5 to 7 carbon atoms, disaccharides composed of hexose, sugar alcohols with 4 to 6 carbon atoms, ethers formed from monosaccharides with 5 to 7 carbon atoms and monohydric alcohols, and ethers formed from disaccharides composed of hexose and monohydric alcohols, and

C: one or more of alcohols selected from secondary alcohols and tertiary alcohols.

According to the present invention, since an immobilized thermostable lipase is used for reacting the starting materials A and B and the enzymatic reaction is carried out under the presence of the secondary alcohol and/or tertiary alcohol, the reaction can proceed rapidly, by-production of poly substituents such as diacyl compounds, triacyl compounds, etc. is suppressed and saccharid, fatty acid esters with high content of monoester can be obtained as the reaction products, whereby saccharide fatty acid monoesters can be produced at a hgh synthesis ratio with an industrial advantage.

In the present invention, an acyl donor and a saccharide are used as starting materials.

The acyl donor is selected from saturated and unsaturated fatty acids with 6 to 22 carbon atoms, lower alkyl esters of such fatty acids, and glycerol esters of such fatty acids. They are used alone or as a combination of two or more of them.

More specifically, the acyl donor used in the present invention includes a saturated or unsaturated, linear or branched fatty acid having 6 to 22 carbon atoms. One or more of hydrogen atoms in the fatty acid may be substituted with hydroxy groups, carbonyl groups, phenyl groups and like other groups. Examples of the fatty acids usable herein include capric acid, sorbic acid, caprylic acid, caproic acid, lauric acid, myristic acid, palmitoleic acid, palmitic acid, stearic acid, isostearic acid, oleic acid, linolic acid, linolenic acid, pentadecanic acid, eicosanic acid, docosanic acid, docosenic acid, arachidonic acid, ricinoleic acid and dihydroxy stearic acid.

As the ester of the fatty acid, an ester of the fatty acid with 6 to 22 carbon atoms and lower alcohol with 1 to 3 carbon atoms, for example, methanol, ethanol or propanol is used. Specifically, there can be mentioned, for example, methyl capronate, ethyl caprate, methyl laurate, ethyl laurate, propyl laurate, methyl myristate, ethyl myristate, propyl myristate, methyl palmitate, ethyl palmitate, propyl palmitate, methyl stearate, ethyl stearate, propyl stearate, methyl oleate, ethyl oleate, propyl oleate, methyl linolate, ethyl linolate, propyl linolate, methyl linolenate, ethyl linolenate, propyl linolenate, methyl eicosanate, methyl arachidonate, methyl docosanate and methyl docosenate.

As the glycerol ester of the fatty acid, 1,3- diglyceride, 1,2-glyceride or triglyceride of the fatty acd described above is used suitably. In this case, the fatty acid residues in diglycerides or triglycerides may be identical or different with each other. Monoglyceride of the fatty acid can also be used. As the glycerol ester, there can be mentioned, for example, glycerol-1,3-dipalmitate, glycerol-1,2-dioleate, glycerol-1,2-distearate, glycerol-1,3-dilaurate, glycerol tricaprilate, glycerol myristate, palm oil, coconut oil, glycerol monocaprate and glycerol monostearate.

The saccharide used in the present invention is one or more of saccharides selected from monosaccharides with 5 to 7 carbon atoms, disaccharides composed of hexose, sugar alcohols with 4 to 6 carbon atoms, ethers formed from monosaccharides with 5 to 7 carbon atoms and monohydric alcohols, and ethers formed from disaccharides composed of hexose and monohydric alcohols.

As the $C_5$-monosaccharides, there can be mentioned arabinose, ribose, xylose, lyxose, xylulose, rubulose and 2-dexyribose. As $C_6$-monosaccharides, there can be mentioned glucose, galactose, fructose, mannose, sorbose, talose, 2-deoxyglucose, 6-deoxygalactose, 6- deoxymannose, and 2-deoxygalactose. As the $C_7$-monosaccharides, there can be mentioned arohepturose, sedo hepturose, mannohepturose and gluocohepturose.

The disaccharides composed of hexose include maltose, sucrose and sophorose.

The sugar alcohols include erythritol, ribitol, xylytol, allitol, sorbitol, mannitol and galactitol.

As the ethers of $C_{5-7}$ monosaccharides or disaccharides composed of hexose and monohydric alcohols, preferred are those having monohydric alcohols with 1 to 12, particularly, 1 to 6 carbon atoms. The carbon chain of such alcohols may be linear or branched, saturated or unsaturated, and substituted or unsubstituted. Methanol, ethanol, propanol and butanol are preferred as the alcohol.

In this case, there is no particular restriction for the ether bonding position between the saccharides and monohydric alcohol. Specific examples are glycosides such as methyl glucoside, ethyl glucoside, propyl glucoside, butyl glucoside, methyl fructoside, methyl mannoside, methyl maltoside and methyl lactoside or sugar ethers such as 6-O-methyl glucose and 6-O-methyl fructose. As the alkyl glycosides, those having an alkyl group as an aglycone to the hemi-acetal (anomer) hydroxy group are used. In addition, those in which the steric configuration after alkyl substitution for hemi-acetal (anomer) hydroxy groups are present solely for $\alpha$, $\beta$ or at an optional mixing ratio for $\alpha$, $\beta$ may be used.

3

Among the saccharides described above, those used suitably are glucose, fructose, galactose, mannose, maltose, sucrose, sorbitol, methyl glucoside, ethyl glucoside, propyl glucoside, butyl glucoside, methyl fructoside, methyl polyglucoside (maltoside, isomaltoside).

When one or more of saccharides selected from monosaccharides with 5 to 7 carbon atoms, disaccharides composed of hexose and sugar alcohols with 4 to 6 carbon atoms and one or more of ethers of the saccharides described above and monohydric alcohols are used together in admixture, a mixture of fatty acid esters of saccharides and fatty acid esters of saccharide ethers can be synthesized at once in accordance with a mixing ratio and at a high efficiency for aimed products. For the mixture, saccharides (for example, glucose) and a monohydric alcohol (for example, methanol) are reacted by a Helferich's method (Organic Synthesis, Page 336, 1956) to prepare and use a mixture of saccharides (glucose) and saccharide ethers (methyl glucoside). The saccharides and the saccharide ethers can be present at a weight ratio within arange from 98 : 2 to 2 : 98 in the reaction system and they are used being mixed substantially at the same ratio for the desired ester mixture.

The mixing ratio between the acyl donor and the saccharide is preferably from 0. 02 to 50 mol, more preferably from 0.05 to 10 mol of the saccharide based on one mol of the acyl donor.

In this case, saccharide fatty acid monoesters can be obtained selectively and more reliably by using the acyl donor at a molar ratio greater than one mol, preferably, within a range from 1 mol to 50 mol, more preferably, 1.5 to 20 mol and, particularly, 2 to 8 mol to one mol of the saccharide.

Then, in the present invention, an immobilized thermostable lipase is used for reacting the starting materials as described above by utilizing an enzymatic reaction. Various kinds of thermostable lipase can be used so long as they have such heat resistivity as possessing a residual activity of greater than 40% preferably, greater than 80% and, more preferably, greater than 95% after dissolving 50 mg of lipase powder into 0.5 ml of phosphoric acid buffer (0. 1M, pH 7) and heating them at 70 °C for 30 min. For instance, neutral thermostable lipases such as thermostable lipase derived from Candida antarctica (sp-382, manufactured by NOVO Co.) and a thermostable lipase derived from Mucor miehei (Lipozyme, manufactured by NOVO Co.) are suitably used.

The thermostable lipases may be purified or coarse products and, further, drying products of bacteria producing the thermostable lipase (processed, bacterial or resting bacteria) may also be used.

For the method of immobilizing the thermostable lipase, there may be employed any of carrier bonding method, crosslinking method and inclusion method, although the carrier bonding method can be used particularly suitably.

In this case, examples of the immobilizing carrier include inorganic substances such as activated carbon, porous glass, acidic white clay, bleaching clay, kaolinite, alumina, silica gel, bentonite, hydroxy apatite, calcium phosphate and metal oxides, natural polymeric compounds such as starch and glutene, and synthetic polymeric materials such as polyethylene, polypropylene, phenol formal in resin, acrylic resin, anionic exchange resin and cationic exchange resin. Various immobilizing carriers other than those described above may also be used with no troubles so long as they do not hinder the development of the enzymatic activity. Among them, synthetic polymeric material in the physically porous form, for example, porous polyethylene, porous polypropylene, porous phenol formal in resin and porous acrylic resin can be used most preferably.

The lipase is immobilized to the immobilizing carrier preferably in such an amount from 0. 1 to 500 mg of protein content based on one gram of the immobilizing carrier, protein containing about 2 to 80 % by weight of lipase being fixed particularly preferably.

In the present invention, there is no particular restriction to the amount of the immobilized thermostable lipase used, but it can be within a range from 0.0001 to 10,000 parts by weight, preferably, from 0.01 to 2,000 parts by weight based on 100 parts by weight of the acyl donor.

In the present invention, the enzymatic reaction between the acyl donor and the saccharide using the immobilized thermostable lipase is carried out under the presence of one or more of alcohols selected from secondary alcohols and tertiary alcohols.

Examples of the secondary and tertiary alcohols are 2,4-dimethyl-3-pentanol, 2,6-dimethyl-4-heptanol, tertiary butanol, tertiary amyl alcohol, diacetone alcohol, 3-methyl-3-pentanol, 3-ethyl-3-pentanol, 3-propyl-3-pentanol, 2-methyl-2-hexanol and 2-ethyl-2-hexanol.

The amount of the alcohol used varies depending on the kind of the alcohol, the carbon chain length of the fatty acid, the reaction temperature, etc. and it is preferably from 10 to 99% by weight for the entire reaction system.

In addition to the alcohols, there may be used together, if required, aromatic hydrocarbons such as benzene, toluene, xylene and phenol, ketones such as acetone and methyl ethyl ketone, ethers such as diemthyl ether and dioxane, aliphatic hydrocarbons such as n-hexane, n-octane and iso-octane, cycloaliphatic hydrocarbons such as cyclopentane and cyclohexane and halogenated hydrocarbons such as carbon tetrachloride, chloroform and methylene dichloride. Further, nitrogen-containing solvents such as pyridine, dimethylformamide, dimethylacetoamide and quinoline or sulfur-containing solvents such as dimethyl sulfoxide may be used together, which are good solvents to saccharides.

Upon the enzymatic reaction according to the present invention, the saccharides are preferably used by dissolving them into the solvent described above at a temperature higher than 40°C, preferably, 40 to 200°C and, more preferably, 60 to 100°C. As the method, there can be used, for example, a method of dissolving the saccharide to a solvent heated to higher than 40°C, which is then mixed with a mixture of the acyl donor and lipase heated to higher than 40°C, or a method of adding a solvent warmed to higher than 40°C to a mixed system of the acyl donor, saccharide and lipase thereby dissolving the saccharide. In this way, by using the saccharide being dissolved into the solvent at a temperature higher than 40°C and then conducting the reaction at a temperature of higher than 40°C capable of maintaining the dissolved state, the saccharide can be used effectively and saccharide fatty acid monoester can be synthesized at a higher reaction rate and in a shorter period of time.

In a case of conducting an enzymatic reaction for the acyl donor and the saccharide by using the immobilized thermostable lipase, the reaction conditions can properly be adjusted and the reaction can proceed even at a low temperature. However, it is preferred to conduct the reaction at a temperature higher than 40°C, particularly, 50 to 120°C in order to increase the reaction rate. By conducting the reaction under such a temperature condition, the reaction can be completed within about 4 to about 24 hours. In the present invention, the enzyme is not deactivated at such a high temperature since the immobilized thermostable lipase is used.

When saccharide fatty acid monoesters are prepared by the process according to the present invention, the process can be carried out by a method of passing a substrate solution through the immobilized lipase packed in a column (packed column method), a method of introducing the substrate solution and the immobilized lipase into a reaction vessel and conducting the reaction by stirring and shaking (batchwise method), or a method of continuously conducting the reaction in the batchwise method (continuous stirring vessel method).

Since, water or lower alcohol with 1 to 3 carbon atoms, etc. are by-produced in the process according to the present invention through the enzymatic reaction, it is preferred to remove the by-products such that the concentration- of the by-products in the system is less than 0.5% by weight, particularly, less than 0.1% by weight in order to proceed the reaction efficiently. As a method of removing the by-products, there can be mentioned, for example, removing them through adsorption by using an adsorption agent such as zeolite, molecular sieve, sodium sulfate, etc. at the outside and/or the inside of the reaction system, introducing dry air or inert gas into the reaction vessel thereby evaporizing them to remove them into the gas, or reducing the pressure in the reaction vessel and evaporizing and discharging them out of the reaction vessel. The synthesis reaction can be conducted efficiently by properly combining such a removing method with the enzymatic reaction device.

It is recommended that the reaction is terminated then the reaction rate reaches 60 to 99% and the reaction mixture is filtered to separate the unreacted starting saccharide and the immobilized lipase.

The reaction rate nay be defined to preferably 60 to 99% so that the lipase can be used stably and repeatedly for a long pericd of time (batchwise reaction) or used continuously by leaving the unreacted saccharide in the reaction system and utilizing the enzyme stabilizing effect of saccharide. That is, the reaction rate per hour is most high till it reaches 60 to 99%, in which the enzyme can be utilized most efficiently when considering the enzyme durability.

When the reaction mixture is filtered to collect the saccharide and the lipase, it is preferred that the reaction mixture is cooled to room temperature or to a further lower temperature and they are filtered after sufficiently depositing the saccharide. The filtration can be conducted in accordance with a customary method.

Since the saccharides are less soluble to the organic solvent of the reaction system under room temperature, it has to be warmed in a case of dissolving them into the organic solvent. Accordingly, when the temperature of the reaction mixture is cooled to room temperature or lower after the reaction proceeds to 60 to 99%, the saccharide is readily deposited and can be recovered by filtration, while the filtered lipase suffers from no reduction in the enzymatic activity and can be used effectively to other reaction system.

Thus, the saccharide and lipase filtered and separated from the reaction mixture can be re-used as they are and added to the new reaction system for repeating the reaction described above. In this case, an additional saccharide and lipase can be supplemented separately.

By the process as described above the saccharade and the lipase can be recovered for reuse reliably and simply to efficiently synthesize a saccharide fatty acid monoester with an economical advantage.

The saccharide may be removed by treating the reaction mixture with water. Accordingly, after separating the saccharide and the immobilized lipase by the filtration as described above, a small amount of saccharide remaining in the reaction mixture can be transferred into an aqueous phase and removed by treating the reaction mixture after distilling off the solvent with water. The saccharide transferred to the aqueous phase can be dried and then used again together with the saccharide separated and recovered by the filtering treatment described above.

The saccharide can of course be separated also by treating the reaction mixture with water before filtration, thereby transferring the unreacted saccharide in the reaction mixture into the aqueous phase.

The water treatment in this case is applied after distilling off the organic solvent used if it is water-soluble. Alternatively, if the solvent is water-insoluble, the water treatment is applied as it is without distilling off the organic solvent or after distilling off the solvent as required. As the treating method, any of appropriate means such as extraction, washing, etc. can be used alone or in combination. Further, the amount of water used is properly selected and it is usually from 2 to 50 times by weight to the solid content in the reaction mixture. Although the treatment can be applied sufficiently at room temperature, efficient treatment can be attained by using warm water heated to 40 to 80° C.

There is no particular restriction to the method of recovering a saccharide fatty acid ester from the reaction mixture, but it is effective to treat the reaction mixture with an organic solvent which is capable of dissolving the acyl donor but incapable of dissolving the saccharide fatty acid ester to separate them into the acyl donor and the saccharide fatty acid ester.

As the organic solvent capable of dissolving the acyl donor but incapable of dissolving the saccharide fatty acid ester, there can be mentioned saturated hydrocarbons such as n-pentane, n-hexane, n-heptane, 2-methylheptane, 2-octane and iso-octane, unsaturated hydrocarbons such as 2-hexene and 2-octene, aromatic hydrocarbons such as benzene, toluene and xylene and ketones such as acetone and methyl ethyl ketone. The acyl donor is recovered by being transferred into the solvent.

The treatment with the organic solvent can be applied after distilling off or without distilling off the organic solvent in the reaction mixture depending on the case. As the treating method, appropriate means such as washing, extraction, column method or recrystallization may be used alone or in combination. The amount of the organic solvent used may be selected properly but it is usually from 2 to 50 times (by weight ratio) based on the solid content in the reaction mixture. Although the treatment can be applied sufficiently even at room temperature, a warmed organic solvent can be used and treatment is also applied at the refluxing temperature of the organic solvent.

The acyl donor thus separated by the treatment of the organic solvent can be recovered and used again for the reaction.

The saccharide fatty acid ester from which the acyl donor is separated contains the monoester at high content and, accordingly, it can be used as it is. Depending on the purpose, it may be purified by means of re-crystallization, column method or crystallization and can be separated into monoester and polyester such as diester and triester. In this case, since the acyl donor is separated, purification can be applied easily.

Accordingly, the saccharide fatty acid monoester can be recovered efficiently by properly combining the separation and recovery methods for enzyme, saccharide and acyl dcnor.

EXPERIMENTAL EXAMPLES

Thermostability Test for Various Lipases

After dissolving 50 mg of a powder for each oF various lipases shown in Table I in 4 ml phosphoric acid buffer (0.1M, pH 7), it was heated at 70° C for 30 min. After cooling it by cold water, it was dissolved in 5g of glycerol and enzymatic reaction was conducted at 30° C for one hour with addition of 0.5g of oleic acid. After the reaction was over, 20 ml of an acetone:ethanol solution (1:1) (by volume ratio) was added to terminate the enzymatic reaction. Further, a not-heated aqueous solution of lipase was used as a blank and enzymatic reactions were conducted in parallel.

After terminating the enzymatic reaction, it was titrated with 0. 1M ethanol - potassium hydroxide solution to calculate the enzymatic activity based on the amount of the fatty acid decreased and the relative activity before and after the heating was determined by using the following equation:

6

$$\begin{matrix} \text{Relative} \\ \text{activity} \\ (\%) \end{matrix} = \dfrac{\text{Enzymatic activity}}{\text{after heating}} \times 100$$

Table I

| Lipase | | Relative Activity (%) |
|---|---|---|
| Origin | Commercial Name (Manufacturer's Name) | |
| Candida antarctica | sp-382 (NOVO Co., Ltd.) | 98 |
| Mucor sp | sp-225 (NOVO Co., Ltd.) | 0 |
| Mucor sp | sp-356 (NOVO Co., Ltd.) | 0 |
| Mucor sp | sp-400 (NOVO Co., Ltd.) | 0 |
| Rhizopus delemer | Lipase (Seikagaku Kogyo Co., Ltd.) | 6 |
| - | Lipase (Nagase Industrial Co., Ltd.) | 0 |
| - | Lipase (Toyo Jozo Co., Ltd.) | 0 |
| - | Talipase (Tanabe Seiyaku Co., Ltd.) | 0 |
| Candida cylindracea | Lipase MY (Meito Sangyo Co., Ltd.) | 0 |
| Candida cylindracea | Lipase OF (Meito Sangyo Co., Ltd.) | 3 |
| Achromobacter sp | Lipase AL (Meito Sangyo Co., Ltd.) | 15 |
| Alcaligenes sp | Lipase PL (Meito Sangyo Co., Ltd.) | 11 |
| Pseudomonas fluorescens | Lipase P (Amano Seiyaku Co., Ltd.) | 8 |
| Penicillium aurantiogriseum | Lipase G (Amano Seiyaku Co., Ltd.) | 0 |
| Aspergillus niger | Lipase AP (Amano Seiyaku Co., Ltd.) | 0 |
| Rhizopus sp | Saiken (Osaka Saikin Laboratory) | 0 |
| Rhizopus japonicus | Olipase (Osaka Saikin Laboratory) | 0 |
| Arthrobacter sp | BSL (Godo Shusei Co., Ltd.) | 0 |
| Rhizopus arrhizus | Lipase (Sigma Co., Ltd.) | 0 |
| Porcine pancreas | Lipase (Sigma Co., Ltd.) | 0 |

The present invention will now be explained specifically referring to examples and comparative examples but it should be understood that the present invention is not restricted to the following examples.

[Example 1]

To a mixture of 5.00 g (27.65 mM) of glucose and 1. 03 g (5.55 mM) of methyl caprate, 25 ml of tertiary butyl alcohol added and 100 mg of thermostable lipase derived from Candida antarctica (hereinafter referred to as immobilized lipase sp-382) were further added and, subsequently, 10 g of molecular sieves 5A was added as the methanol remover and it was refluxed under heating while stirring for 24 hours.

Then, 0.5 ml of the reaction solution was taken into a 5 ml screw tube, to which 2.5 ml of pyridine and, further, 10 ml of n-tetradecane as an internal standard substance were added. After sufficiently mixing them, they were filtered and 1 ml of anhydrous acetic acid was added as an acetylating agent to 1 ml of the filtrate and reacted at 60° C for 30 min.

1 μl of the reaction solution was analyzed on gas chromatography to measure the weight percent of the resultant glucose fatty acid ester.

As a result, glucose monocaprate was obtained at 98% purity and 70% yield.

[Examples 2 to 8]

Saccharide fatty acid esters were prepared in the same procedure as those in Example 1 by using the starting materials shown in Table II and under the conditions also shown in Table II. The results are shown in the Table.

Table II-1

| Example | Saccharide | Acyl donor | Enzyme | Solvent | Molecular sieves | Reaction conditions | Purity of saccharide monoester | Yield of saccharide monoester |
|---|---|---|---|---|---|---|---|---|
| 1 | Glucose 5.15 g (27.64mM) | Methyl caprate 1.03 g (5.5mM) | sp-382 (NOVO) 100 mg | Tertiary butyl alcohol 25 ml | 5A 10 g | Reflux for 24 hours | 98% | 70% |
| 2 | Glucose 1.00 g (5.55mM) | Ethyl caprate 1.11 g (5.55mM) | sp-382 (NOVO) 100 mg | Tertiary butyl alcohol 25 ml | 5A 10 g | Reflux for 24 hours | 96% | 65% |
| 3 | Glucose 5.15 g (27.64mM) | Capric acid 0.96 g (5.55mM) | sp-382 (NOVO) 100 mg | Tertiary butyl alcohol 25 ml | 3A 10 g | Reflux for 24 hours | 97% | 60% |
| 4 | Fructose 5.15 g (27.64mM) | Methyl caprate 1.03 g (5.55mM) | sp-382 (NOVO) 100 mg | Tertiary butyl alcohol 25 ml | 5A 10 g | Reflux for 24 hours | 95% | 60% |

EP 0 413 307 A1

EP 0 413 307 A1

Table II-2

| Example | Saccharide | Acyl donor | Enzyme | Solvent | Molecular sieves | Reaction conditions | Purity of saccharide monoester | Yield of saccharide monoester |
|---------|------------|------------|--------|---------|------------------|---------------------|-------------------------------|-------------------------------|
| 5 | Sucrose 9.50 g (27.75mM) | Methyl caprate 1.03 g (5.55mM) | sp-382 (NOVO) 100 mg | Tertiary butyl alcohol 25 ml | 5A 10 g | Reflux for 24 hours | 95% | 70% |
| 6 | Glucose 5.15 g (27.64mM) | Methyl laurate 1.19 g (5.55mM) | sp-382 (NOVO) 100 mg | Tertiary amyl alcohol 25 ml | 5A 10 g | Reflux for 24 hours | 96% | 60% |
| 7 | Glucose 5.15 g (27.64mM) | Methyl oleate 1.65 g (5.55mM) | sp-382 (NOVO) 100 mg | Tertiary butyl alcohol 25 ml | 5A 10 g | Reflux for 24 hours | 97% | 65% |
| 8 | Glucose 5.15 g (27.64mM) | Methyl caprylate 0.88 g (5.55mM) | sp-382 (NOVO) 100 mg | Tertiary butyl alcohol 25 ml | 5A 10 g | Reflux for 24 hours | 98% | 70% |

[Comparative Example 1]

1 gram of fructose, 6 g of oleic acid, 70 mg of lipase Al powder (Achromobacter sp.) and 15 ml of tertiary butyl alcohol were charged in a 50 ml volume Erlenmeyer flask and reciprocally shaken at 30°C for 24 hours. The reaction solution centrifugally removed from insoluble matter was concentrated in a rotary evaporator to remove the solvent. After dissolving the residue by addition of 20 ml of chloroform, insoluble matter was centrifugally removed. When the supernatant was separated by column, 410 mg of a mixture of fructose monooleate and fructose dioleate at 5:1 mixing ratio was obtained.

[Example 9]

100 g (0.56 mol) glucose were dissolved in 2.5 liters of tertiary butyl alcohol at 70°C, to which 515 g (2.78 mol) of methyl caprate were added and, further, 10 g of thermostable lipase derived from Candida antarctica immobilized to an acrylic resin (immobilized lipase sp-382, manufactured by NOVO Co.) were added. Subsequently, they were refluxed under heating while stirring for 24 hours by using 500 g of molecular sieves 5A as a methanol removing agent.

Then, 0.5 ml of the reaction solution was charged into a 5 ml screw tube, to which 2.5 ml of pyridine and, further, 10 $\mu$l of n-tetradecane as the internal standard substance were further added and, after sufficiently mixing, they were filtered and 1 ml of anhydrous acetic acid was added as an acetylating agent to 1 ml of the filtrate and reacted at 60°C for 30 min.

1 $\mu$l of the reaction solution was analyzed on gas chromatography and the weight percent of the resultant fatty acid ester of glucose was measured.

As a result, glucose monocaprate was obtained at 98% purity and 95% yield.

[Examples 10 to 17]

Saccharide fatty acid esters were prepared in the same procedures as those in Example 9 by using the starting materials and under the conditions as shown in Table III. The results are shown in the table.

Table III-1

| Example | Saccharide | Acyl donor | Enzyme | Solvent (Saccharide dissolving temperature) | Molecular sieves | Reaction conditions | Purity of saccharide monoester | Yield of saccharide monoester |
|---|---|---|---|---|---|---|---|---|
| 10 | Glucose 100 g (0.56 mol) | Methyl caprate 515 g (2.78 mol) | sp-382 10 g | Tertiary butyl alcohol 2.5ℓ (70℃) | 5A 500 g | Reflux for 24 hours | 98% | 95% |
| 11 | Glucose 1.00 g (5.55mM) | Ethyl caprate 5.55 g (27.75mM) | sp-382 100 mg | Tertiary butyl alcohol 25mℓ (70℃) | 5A 10 g | Reflux for 24 hours | 95% | 92% |
| 12 | Fructose 1.00 g (5.55mM) | Methyl Caprate 3.09 g (16.65mM) | sp-382 100 mg | Tertiary butyl alcohol 25mℓ (70℃) | 5A 10 g | Reflux for 24 hours | 93% | 96% |
| 13 | Glucose 1.00 g (5.55mM) | Capric acid 4.80 g (27.75mM) | sp-382 100 mg | Tertiary butyl alcohol 25mℓ (70℃) | 3A 10 g | Reflux for 24 hours | 97% | 94% |

EP 0 413 307 A1

Table III-2

| Example | Saccharide | Acyl donor | Enzyme | Solvent (Saccharide dissolving temperature) | Molecular sieves | Reaction conditions | Purity of saccharide monoester | Yield of saccharide monoester |
|---|---|---|---|---|---|---|---|---|
| 14 | Glucose 1.00 g (5.55mM) | Methyl laurate 5.95 g (27.75mM) | sp-382 100 mg | Tertiary butyl alcohol 25mℓ (70℃) | 5A 10 g | Reflux for 24 hours | 93%. | 94% |
| 15 | Glucose 1.00 g (5.55mM) | Methyl oleate 8.25 g (27.75mM) | sp-382 100 mg | Tertiary amyl alcohol 25mℓ (70℃) | 5A 10 g | 24 hours 80℃ | 96% | 92% |
| 16 | Glucose 1.00 g (5.55mM) | Methyl caprilate 4.40 g (27.75mM) | sp-382 100 mg | Diacetone alcohol 25mℓ (70℃) | None (Methanol removed under reduced pressure) | 24 hours 80℃ | 97% | 96% |

EP 0 413 307 A1

[Example 17]

To a mixture of 10.0 g (55.5 mM) of glucose and 51.5 g (276.4 mM) of methyl caprate, 250 ml of tertiary butyl alcohol and 1 g of thermostable lipase derived from Candida antarctica immobilized to an acrylic resin (immobilized lipase sp-392, manufactured by NOVO Co.) were added and, subsequently they were refluxed under heating while stirring for 8 hours by using 100 g of molecular sieves 5A as a methanol removing agent.

After cooling the reaction mixture to room temperature, unreacted glucose and immobilized lipase were recovered by filtration.

When solid matters obtained by distilling off the solvent from the fractionated liquid filtrate were washed with n-hexane and filtered 12.0 g of white powder of glucose caprate were obtained as the filtration residue.

The reaction solution was analyzed on gas chromatography and the weight percent of the resultant glucose fatty acid ester was measured. As a result, glucose monocaprate was obtained at 98% purity and 85% yield.

To the thus recovered 1 g of immobilized lipase and 3 g of glucose, 7.0 g (38.9 mM) of glucose and 51.5 g (276.4 mM) of methyl caprate were further added and they were refluxed under heating for 8 hours while removing methanol by using molecular sieves 5A (100 g). As a result of analyzing the aimed products on gas chromatography, glucose monocaprate was obtained at 97% purity and at 70% yield.

When the same procedure was repeated five times, the following results (purity and yield of glucose monocaprate) were obtained for the respective times.

Third process 97% purity 68% yield
Fourth process 98% purity 73% yield
Fifth process 96% purity 72% yield


[Examples 18 to 20]

Saccharide fatty acid esters were prepared in the same procedures as those in Example 17 by using the starting materials and under the conditions as shots in Table IV. The results are shown in the table.

Table IV

| Example | | Saccharide | Acyl donor | Enzyme | Solvent | Molecular sieves | Reaction condition | Purity of saccharide monoester | Yield of saccharide monoester |
|---|---|---|---|---|---|---|---|---|---|
| 18 | First reaction | Glucose 10.0 g (55.5mM) | Methyl caprate 51.5 g (276.9mM) | sp-382 1 g | Tertiary butyl alcohol 250mℓ | 5A 100 g | Reflux for 8 hours | 98% | 65% |
| | Second reaction | Recovered (3.0 g) 7.0g (Added) (38.9mM) | 51.5 g | Recovered (1 g) | Tertiary butyl alcohol 250mℓ | 5A 100 g | Reflux for 8 hours | 98% | 70% |
| 19 | First reaction | Glucose 10.0 g (55.5 mM) | Capric acid 48.0 g (277.5 mM) | sp-382 1 g | Tertiary butyl alcohol 250mℓ | 5A 100 g | Reflux for 8 hours | 95% | 70% |
| | Second reaction | Recovered (2.5 g) 7.5 g (Added) (41.7mM) | 48.0 g | Recovered (1 g) | Tertiary butyl alcohol 250mℓ | 5A 100 g | Reflux for 24 hours | 96% | 96% |
| 20 | First reaction | Fructose 10.0 g (55.5 mM) | Methyl laurate 59.4 g (277.5mM) | sp-382 1 g | Tertiary butyl alcohol 250mℓ | 5A 100 g | Reflux for 6 hours | 94% | 75% |
| | Second reaction | Recovered (2.0 g) 8.0 g (Added) (44.4mM) | 59.4 g | Recovered (1 g) | Tertiary butyl alcohol 250mℓ | 5A 100 g | Reflux for 24 hours | 97% | 94% |

[Example 21]

To a mixture of 5.00 g (27.75 mM) of glucose and 1.03 g (5.55 mM) of methyl caprate, 25 ml of tertiary butyl alcohol and 100 mg of thermostable lipase derived from Candida antarctica and immobilized to an acrylic resin (immobilized lipase) were further added and, subsequently, they were refluxed under heating while stirring for 24 hours by using 10 g of molecular sieves 5A as a methanol removing agent.

The reaction solution was analyzed on gas chromatography to measure the weight percent of the resultant glucose fatty acid ester. As a result, glucose monocaprate was obtained at 98% purity and at 70% yield.

Then, after cooling the reaction mixture to room temperature, the tertiary butyl alcohol was distilled off. After washing the resultant residue with 100 ml of water at 40°C and filtering the washing liquid, the filtrate was dried to obtain 4.2 g of white powder of glucose. A mixture of the immobilized lipase, methyl caprate and glucose caprate obtained as the filtration residue was dried and washed with n-hexane to obtain a mixture of immobilized lipase and glucose caprate. Further, when n-hexane was distilled off from the liquid filtrate, 0.3 g of methyl caprate was recovered.

Next, when the filtration residue was washed and filtered with methanol, glucose caprate was obtained as a liquid filtrate and when methanol was distilled off from the liquid filtrate, 1.26 g of white powder of glucose caprate was obtained.

[Example 22]

To a mixture of 5.00 g (27.75 mM of glucose and 1.03 g (5. 55 mM) of methyl caprate, 25 ml of tertiary butyl alcohol and 100 mg of thermostable lipase derived from Candida antarctica and immobilized to an acrylic resin (immobilized lipase) were further added and, subsequently, they were refluxed under heating while stirring for 24 hours by using 10 g of molecular sieves 5A as a methanol removing agent.

The reaction solution was analyzed on gas chromatography to measure the weight percent of the resultant glucose fatty acid ester. As a result, glucose monocaprate was obtained at 98% purity and 70% yield.

Then, after cooling the reaction mixture to room temperature, it was filtered and the immobilized lipase and the unreacted glucose were recovered. The solvent was distilled off from the tertiary butyl alcohol solution of a mixture of methyl caprate and glucose caprate obtained as the liquid filtrate. The resultant mixture of methyl caprate and glucose caprate was dried and, when it was washed with n-hexane, methyl caprate was leached into n-hexane to obtain 1.26 g of a white powder of glucose caprate as the residue. It was washed with 10 ml of water to remove glucose mixed in a slight amount. Further, when n-hexane was distilled off from the liquid filtrate, 0.3 g of methyl caprate was recovered.

[Example 23]

To a mixture of 5.00 g (27.75mM) of glucose and 1.03 g (5.55 mM) of methyl caprate, 25 ml of tertiary butyl alcohol and 100 mg of thermostable lipase derived from Candida antarctica and immobilized to an acrylic resin (immobilized lipase) were further added and, subsequently, they were refluxed under heating while stirring for 24 hours by using 10 g of molecular sieves 5A as a methanol removing agent.

The reaction solution was analyzed on gas chromatography to measure the weight percent of the resultant glucose fatty acid ester. As a result, glucose monocaprate was obtained at 98% purity and at 70% yield.

Then, after cooling reaction mixture to room temperature, it was filtered and the immobilized lipase and the unreacted glucose were recovered. The solvent was distilled off from the tertiary butyl alcohol solution of a mixture of methyl caprate and glucose caprate obtained as the liquid filtrate. The resultant mixture of methyl caprate and glucose caprate ester was dried and, when it was washed with n-hexane, methyl caprate was leached into n-hexane to obtain 1.26 g of white powder of glucose caprate as the residue. When n-hexane was distilled off from the solution, 0.3 g of methyl caprate was recovered. After separating 1 g from 1.26 g of the white powder of the resultant glucose caprate and dissolving it into 20 ml of a mixed solvent of acetone : methanol (= 4:1) under heating (on a water bath), it was cooled to 5°C to obtain 0.68 g of glucose monocaprate crystals.

The foregoing reaction was further repeated for five times and, as a result of washing with n-octane, 2-hexene, toluene and acetone instead of n-hexane, similar results were obtained respectively.

[Example 24]

1 g (5.2 mM) of methyl glucoside, 6 g (21.3 mM) of oleic acid, 100 mg of neutral thermostable lipase derived from Candida antarctica and immobilized to an acrylic resin (immobilized lipase sp-382, manufactured by NOVO Co.), 2 g of molecular sieves 5A as a methanol removing agent and 15 ml of tertiary butyl alcohol were charged into a 50 ml volume Erlenmeyer flask and shaken reciprocally at 60°C for 24 hours to conduct esterifying reaction.

After the reaction, the reaction solution was concentrated to recover the enzyme, while the liquid filtrate was concentrated in a rotary evaporator to remove the solvent. When the residue was separated on column chromatography, methyl-6-O-oleyl glucoside was obtained at 96% yield. Further, the forming ratio between the monoester and the diester was 99.5/0.5.

[Example 25]

19.4 g (0.1 mol) of methyl glucoside and 93.0 g of (0.5 mol) of methyl n-decanoate were added to 150 g of t-amyl alcohol, to which 2 g of immobilized neutral thermostable lipase (Lipozyme, manufactured by NOVO Co.) were added and, further, 30 g of molecular sieves 5A were added as a methanol removing agent and, subsequently, they were reacted at 40°C for 24 hours.

After the reaction, the reaction solution was acetylated by a customary method and, when analyzed on gas chromatography, it was confirmed that methyl-6-O-decanoyl glucoside was obtained at 96% yield and at 99.5% selectivity.

[Example 26]

19.4 g (0.1 mol) of methyl glucoside and 50.5 g (0.3 mol) of n-decanoic acid were added to 120 g of diacetone alcohol and, after adding immobilized neutral thermostable lipase sp-382, they were reacted at 40°C under a reduced pressure (30 mmHg) for 24 hours.

After the reaction, the reaction solution was acetylated by a customary method and when it was analyzed on gas chromatography, it was confirmed that methyl-6-O-decanoyl glucoside was obtained at 97% yield and at 99.3% selectivity.

[Comparative Example 2]

Esterifying reaction was conducted in the same procedures as those in Example 24 except for using 100 mg of powder of an alkaline lipase PL-679 instead of the immobilized neutral thermostable lipase.

20 ml of chloroform were added to the residue obtained by the same procedure for the reaction solution as in Example 24, they were dissolved and then insoluble matters were centrifugally removed. When the supernatant was separated on column chromatography, it was confirmed that methyl-6-O-oleyl glucoside was obtained but the yield was 258 mg (as low as 11%).

[Example 27]

1.04 g (5 mM) of ethyl glucoside and 1.8 g (11 mM) of methyl octanoate were added to 100 ml of tertiary butyl alcohol and ester exchange reaction was conducted at 60°C for 24 hours under stirring while using 0.5 g of immobilized neutral thermostable lipase sp-382.

After the reaction, the enzyme was removed, the solvent was distilled off under a reduced pressure and the residue was separated on column chromatography, thereby obtaining ethyl-6-O-octanoyl glucoside at 98% yield. The forming ratio between the monoester and the diester was 99.7/0.3.

[Comparative Example 3]

1.04 g (5 mM) of ethyl glucoside, 1.8 g (11 mM) of methyl octanoate were added to 100 ml of 2-butanone and ester exchange reaction was conducted by using 0.5 g of a neutral immobilized lipase (Lipozyme, manufactured by NOVO Co.) while stirring at 60°C for 24 hours.

Then, when the same procedures were applied as those in Example 27, it was confirmed that ethyl-6-O-octanoyl glucoside was obtained but the yield was as low as 10%.

[Examples 28 to 33]

Various fatty acid monoesters of alkyl glycosides were prepared by using the starting materials and under the conditions as shown in Table V.

Table V

| Example | Alkyl glucoside | Acyl donor | Enzyme | Solvent | Reaction condition | Yield of alkyl ester (di/mono) |
|---|---|---|---|---|---|---|
| 28 | Butyl glucoside 1.18 g (5 mM) | Decanoic acid 5.04 g (15 mM) | Lipozyme (NOVO Co.) 0.5 g | Tertiary amyl alcohol 100 ml | 50°C under stirring, using molecular sieves (10 g), 24 hours | Butyl-6-0-decanoyl glucoside 95% (0.5/99.5) |
| 29 | Propyl glucoside 1.18 g (5 mM) | Methyl octanoate 1.56 g (10 mM) | sp-382 (NOVO Co.) 0.5 g | Di-acetone alcohol 100 ml | 60°C, stirring at reduced pressure, 24 hours | Propyl-6-0-octanoyl glucoside 96% (0.3/99.7) |
| 30 | Ethyl fructoside 1.04 g (5 mM) | Methyl laurate 3.12 g (15 mM) | sp-382 (NOVO Co.) 0.5 g | Di-acetone alcohol 100 ml | 50°C under stirring, using molecular sieves (10 g), 24 hours | Ethyl-6-0-lauroyl fructoside 97% (0.3/99.7) |
| 31 | Ethyl lactoside 1.85 g (5 mM) | Lauric acid 1.94 g (10 mM) | Lipozyme (NOVO Co.) 0.5 g | Tertiary butyl alcohol 100 ml | 50°C under stirring, using molecular sieves (10 g), 24 hours | Ethyl-lauroyl glucoside 98% (0.5/99.5) |
| 32 | Propyl glucoside 1.11 g (5 mM) | Methyl octanoate 1.56 g (10 mM) | Lipozyme (NOVO Co.) 0.5 g | Tertiary amyl alcohol 100 ml | 60°C under stirring, using molecular sieves (10 g), 24 hours | Propyl-6-0-octanoyl fructoside 97% (0.3/99.7) |
| 33 | Butyl glucoside 1.18 g (5 mM) | Decanoic acid 0.84 g (5 mM) | sp-382 (NOVO Co.) 0.5 g | Di-acetone alcohol 100 ml | 60°C under stirring, using molecular sieves (10 g), 24 hours | Butyl -6-0-decanoyl glucoside 98% (0.2/99.8) |

[Example 34]

A mixture of 19.4 g (0.1 mol) of methyl glucoside, 18.0 g (0. 1 mol) of glucose, 186 g (1 mol) of methyl decanoate and 18.5 g of immobilized thermostable lipase sp-382 were added to 200 g of tertiary butyl alcohol and then they were stirred at 60°C for 24 hours while adding 50 g of molecular sieves 5A as a methanol removing agent.

After the reaction, the reaction solution was acetylated by a customary method and the yield was measuredon gas chromatography. As a result, it was confirmed that methyl glucoside decanoate ester was formed at 98% (diester/monoester forming ratio = 1/99), glucose decanoate was formed at 99.6% (diester/monoester forming ratio = 2/98) and that the reaction mixture was obtained at a rate substantially corresponding to the mixing ratio of the starting methyl glucoside and glucose.

After removing enzyme from the reaction mixture by filtration, the solvent was distilled off and the resultant white solid was purified by washing with n-hexane and removing unreacted methyl decanoate.

[Examples 35 to 39]

Ester mixtures were prepared in the same procedures as those in Example 34 while using starting materials and under the same conditions as those shown in Table VI. The results were shown in the table.

Table VI

| Example | Saccharide | Fatty acid | Enzyme | Solvent | Reaction condition | Product forming ratio (diester/monoester) |
|---|---|---|---|---|---|---|
| 35 | Ethyl glucoside 0.5 mol (109 g) Glucose 0.2 mol (36 g) | Methyl octanoate 1.0 mol (156 g) | sp-382 10 g | Tertiary butyl alcohol 500 g | 60°C, 400 rpm under reduced pressure, 24 hours | Ethyl glucoside octanoate 94% (0.5/99.5) Glucose octanoate 96% (1/99) |
| 36 | Methyl fructoside 0.1 mol (19.4 g) Fructose 0.9 mol (162 g) | Glycerol tripalmitate 2.0 mol (1614 g) | sp-382 | Di-acetone alcohol 200 g | 40°C, 400 rpm under reduced pressure, 24 hours | Methyl fructoside palmitate 92% (0.4/99.6) Fructose palmitate 95%(3/97) |
| 37 | Methyl mannoside 0.2 mol (39 g) Mannose 0.8 mol (114 g) | Decanoic acidate 1.5 mol (252 g) | Lipozyme 2 g | Tertiary butyl alcohol 300 g | 80°C, 400 rpm, Molecular sieves 5A, 100 g, 24 hours | Methyl mannoside decanoate 93% (0.3/99.6) Mannose decanoate 96%(2/98) |
| 38 | Methyl maltoside 0.1 mol (36 g) Maltose(hydrate) 0.1 mol (36 g) | Methyl laurate 3.0 mol (624 g) | Lipozyme 2 g | Tertiary amyl alcohol 400 g | 80°C, 400 rpm, Molecular sieves 5A, 50 g, 24 hours | Methyl maltoside laurate 97% (0.2/99.8) Maltose laurate 95% (2/98) |
| 39 | Ethyl lactoside 0.7 mol (259 g) Lactose(hydrate) 0.3 mol (108 g) | Lauric acid 2.0 mol (386 g) | sp-382 50 g | Tertiary amyl alcohol 400 g | 70°C 400 rpm, under reduced pressure, 24 hours | Ethyl lactoside laurate 96% (0.4/99.6) Lactose Laurate 93% (1/99) |

## Claims

1. A process for producing a saccharide fatty acid monoester comprising reacting an immobilized thermostable lipase on a mixture comprising:

(A) one or more of acyl donors selected from saturated and unsaturated fatty acids with 6 to 22 carbon atoms, lower alkyl esters of said fatty acids and glycerol esters of said fatty acids,

(B) one or more of saccharides selected from monosaccharides with 5 to 7 carbon atoms, disaccharides composed of hexose, sugar alcohols with 4 to 6 carbon atoms, ethers of monosaccharides with 5 to 7 carbon atoms and monohydric alcohols and ether compounds of disaccharides composed of hexose and monohydric alcohols, and

(C) one or more of alcohols selected from secondary alcohols and tertiary alcohols.

2. The process of claim 1 wherein the saccharide is selected from glucose, fructose, galactose, mannose, maltose, sucrose, sorbitol, methyl glucoside, ethyl glucoside, propyl glucoside, butyl glucoside, methyl fructoside and methyl polyglucoside.

3. The process of claim 1 wherein one or more of saccharides selected from monosaccharides with 5 to 7 carbon atoms, disaccharides composed of hexose and sugar alcohols with 4 to 6 carbon atoms and one or more of ethers of the saccharides and monohydric alcohols are used together in admixture.

4. The process of claim 1 wherein the molar ratio of the acyl donor and the saccharide is from 1:0.02 to 1:50.

5. The process of claim 1 wherein the acyl donor is used within a range from one mol to 50 mol to one mol of the saccharide.

6. The process of claim 1 wherein the theremostable lipase has such thermostability as possessing a residual activity of greater than 40%, preferably, greater than 80% and, more preferably, greater than 95% after dissolving 50 mg of lipase powder into 0.5 ml of phosphoric acid buffer (0.1M, pH 7) and heating them at 70°C for 30 min.

7. The process of claim 1 wherein the secondary and tertiary alcohols are selected from 2,4-dimethyl-3-pentanol, 2,6-dimethyl-4-heptanol, tertiary butanol, tertiary amyl alcohol, diacetone alcohol, 3-methyl-3-pentanol, 3-ethyl-3-pentanol, 3-propyl-3-pentanol, 2-methyl-2-hexanol and 2-ethyl-2-hexanol.

8. The process of claim 1 wherein the secondary or tertiary alcohol is used in an amount of from 10 to 99% by weight for the reaction system.

9. The process of claim 1 wherein the enzymatic reaction is conducted at a temperature of from 40°C to 200°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | WO-A-8 901 480 (NOVO INDUSTRI A/S) <br> * page 5, lines 17 - 33 * * page 8, line 12 - page 9, line 4 * * page 14, examples 9 and 11 * * page 10, lines 18 - 21 * <br> — — — | 1-9 | C 12 P 7/62 <br> C 12 P 19/44 <br> C 12 P 7/64 <br> C 12 P 19/02 |
| Y | FR-A-2 617 501 (SOCIETE NATIONALE ELF AOUITAINE) <br> * The whole document, especially page 5, lines 1-20 * <br> — — — | 1-9 | C 12 N 9/20 <br> C 07 H 15/04 |
| A | PATENT ABSTRACTS OF JAPAN vol. 12, no. 181 (C-499)(3028) 27 May 1988, <br> & JP-A-62 289190 (DAI ICHI KOGYO SEIYAKU CO. LTD.) 16 December 1987, <br> * the whole document * <br> — — — | 1-9 | |
| D,A | PATENT ABSTRACTS OF JAPAN vol. 11, no. 132 (C-417)(2579) 24 April 1987, <br> & JP-A-61 268192 (MEITO SANGYO K.K.) 27 November 1986, <br> * the whole document * <br> — — — — — | 1-9 | |

|  |
|---|
| TECHNICAL FIELDS SEARCHED (Int. Cl.5) |
| C 12 P <br> C 12 N <br> C 07 H |

The present search report has been drawn up for all claims

| Place of search | Date of completion of search | Examiner |
|---|---|---|
| The Hague | 15 November 90 | ANDRES S.M. |